# EUROPÄISCHE PATENTANMELDUNG

(11) **EP 1 961 725 A1**
(43) Veröffentlichungstag der Anmeldung: **27.08.2008**
(21) Anmeldenummer: 07103066.2
(22) Anmeldetag: 26.02.2007
(51) Int. Cl.: C07C 33/14, C11B 9/00, A61K 8/34, C07C 255/46

(54) **Riech- und Aromastoffe, ihre Herstellung und Verwendung**

(71) Anmelder: Symrise GmbH & Co. KG, 37603 Holzminden (DE)
(72) Erfinder: Hölscher, Bernd, 37620, Halle (DE)
(74) Vertreter: Eisenführ, Speiser & Partner

(57) **Zusammenfassung**

Die vorliegende Erfindung betrifft Verbindungen der Formel (I) 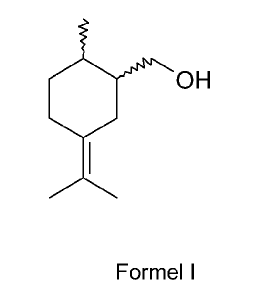 , ihre Verwendungen als Riech- oder Aromastoff, ihre Herstellung, Artikel enthaltend diese Verbindungen und Verfahren zum Vermitteln, Modifizieren und/oder Verstärken eines blumigen und/oder rosigen Geruchs oder Geschmacks.

Die Erfindung betrifft ferner (5-Isopropyliden-2-Methyl-Cyclohexyl)-Carbonitril und Verfahren zu dessen Herstellung. (5-Isopropyliden-2-Methyl-Cyclohexyl)-Carbonitril ist insbesondere geeignet als Zwischenprodukt bei der Herstellung von Verbindungen der Formel (I).

## Beschreibung

Die vorliegende Erfindung betrifft (5-Isopropyliden-2-Methyl-Cyclohexyl)-Methanol, seine Herstellung und seine Verwendung als Riech- und Aromastoff. Die Erfindung betrifft zudem parfümierte oder aromatisierte Artikel umfassend dieser Verbindung sowie entsprechende Verfahren zum Vermitteln, Modifizieren und/oder Verstärken bestimmter Geruchs- und/oder Geschmacksnoten. Die Erfindung betrifft ferner (5-Isopropyliden-2-Methyl-Cyclohexyl)-Carbonitril und Verfahren zu dessen Herstellung.

Trotz einer Vielzahl bereits vorhandener Riechstoffe besteht in der Parfümindustrie auch weiterhin ein genereller Bedarf an neuen Riechstoffen. So besteht ein Bedarf an Rosenriechstoffen mit interessanten Duftnoten in Richtung Pfingstrose, die in der Lage sind, (in Riechstoffkompositionen) neben einer blumigen, rosigen Duftnote weitere interessante Geruchsnoten zu erzeugen und mit ihren neuartigen bzw. originellen Dufteigenschaften die Möglichkeiten des Parfümeurs zu erweitern. Insbesondere besteht ein Interesse an Riechstoffen mit blumigen, rosigen Duftnoten, welche in der Lage sind, eine harmonische Kombination mit holzig duftenden Riechstoffen einzugehen. Vorzugsweise sollte eine Überlagerung der unterschiedlichen geruchlichen Aspekte und Noten erfolgen, um dadurch einen insgesamt komplexen Geruchseindruck zu erzeugen.

Für die Kreation neuartiger moderner Kompositionen besteht ständiger Bedarf an Riechstoffen mit besonderen geruchlichen Eigenschaften, die geeignet sind, als Grundlage für die Komposition von neuartigen modernen Parfüms mit komplexen Geruchscharakter zu dienen. Bevorzugte gesuchte Riechstoffe sollten neben einer blumigen, rosigen Duftnote weitere Noten und Aspekte aufweisen, die ihnen geruchlichen Charakter und Komplexität verleihen.

Die Suche nach geeigneten Riechstoffen, die zur vorliegenden Erfindung führte, wurde durch folgende Sachverhalte erschwert:
- Die Mechanismen der Geruchswahrnehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Daher bestand die dieser Erfindung zugrunde liegende Aufgabe im Wesentlichen darin, Riechstoffe mit blumigen, rosigen Duftnoten zu finden, welche gepaart sind mit weiteren interessanten und originellen geruchlichen Eigenschaften, wodurch die gesuchten Riechstoffe neuartige und originelle Riechstoffkompositionen mit besonderen geruchlichen Noten und Aspekten ermöglichen. Insbesondere sollten Riechstoffe mit blumigen, rosigen Duftnoten gefunden werden, welche insbesondere zur Kombination mit Riechstoffen geeignet sind, welche eine holzige Duftnote aufweisen.

Daneben sollten die diese Hauptaufgabe erfüllenden Riechstoffe ferner vorzugsweise über ihre primären, nämlich geruchlichen, Eigenschaften hinaus zusätzliche positive Sekundäreigenschaften besitzen, wie z.B. eine hohe Stabilität, eine hohe Ausgiebigkeit, ein hohes Haftungsvermögen, eine hohe Substantivität, eine bemerkenswerte Booster-Wirkung oder ein starkes Blooming, so dass sensorisch bemerkenswerte Effekte oder aber auch bessere dermatologische und toxikologische Eigenschaften gegenüber vergleichbaren Riechstoffen erzielt werden können.

Erfindungsgemäß gelöst wird die primäre gestellte Aufgabe durch Angabe der Verbindung (5-Isopropyliden-2-Methyl-Cyclohexyl)-Methanol (Formel I) 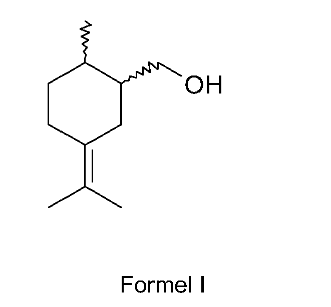 einschließlich ihrer Enantiomeren (1S, 2S)-(Isoproyliden-2-methyl-cyclohexyl)-1-methanol, (1S, 2R)-(Isoproyliden-2-methyl-cyclohexyl)-1-methanol, (1R, 2S)-(Isoproyliden-2-methyl-cyclohexyl)-1-methanol, (1R, 2R)-(Isoproyliden-2-methylcyclohexyl)-1-methanol. Die erfindungsgemäße Verbindung ist insbesondere verwendbar als Riech- oder Aromastoff.

Die erfindungsgemäße Verbindung besitzt zwei chirale Zentren; sie können R- oder S-konfiguriert sein oder als beliebiges Gemisch der Enantiomeren, insbesondere als Racemat eingesetzt werden. Erfindungsgemäß bezeichnet der Begriff "Verbindung der Formel (I)", soweit nichts anderes angegeben, sowohl das Racemat als auch die einzelnen Enantiomere (1S, 2S)-(Isoproyliden-2-methyl-cyclohexyl)-1-methanol, (1S, 2R)-(Isoproyliden-2-methyl-cyclohexyl)-1-methanol, (1R, 2S)-(Isoproyliden-2-methyl-cyclohexyl)-1-methanol, (1R, 2R)-(Isoproyliden-2-methyl-cyclohexyl)-1-methanol und Mischungen zweier oder mehrerer dieser Enantiomeren. Die Enantiomeren können durch Trennung auf einer chiralen Säule oder durch enzymatische Racematspaltung aufgereinigt werden, der Fachmann wird sich dazu an K. Faber, Biotransformations in Organic Chemistry, Springe Verlag , Berlin 1997; C.-H. Wong, G.M. Whitesides, Enzymes in Synthesic Organic Chemistry, Pergamon/Elsevier Science, New York, 1994 orientieren.

Die gestellte Aufgabe wird zudem gelöst durch die Verwendung von (5-Isopropyliden-2-Methyl-Cyclohexyl)-Methanol (Formel I) 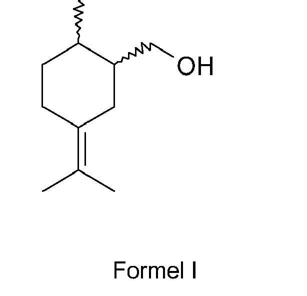 als Riech- oder Aromastoff mit blumigen, rosigen Noten.

Weitere Aspekte ergeben sich aus den beigefügten Patentansprüchen und der folgenden Beschreibung; diese Aspekte betreffen dabei insbesondere neue parfümierte oder aromatisierte Artikel und entsprechende Verfahren.

Die Geruchseigenschaften der erfindungsgemäßen Verbindung (5-Isopropyliden-2-Methyl-Cyclohexyl)-Methanol werden wie folgt beschrieben: sehr schöner strahlender Rosenriechstoff, interessante Duftnote in Richtung Pfingstrose, sehr natürlicher Geruchseindruck. Insbesondere kann die Verbindung zum Vermitteln, Modifizieren und/oder Verstärken einer entsprechenden Geruchs- und/oder Geschmacksnote eingesetzt werden.

Entsprechend dem beschriebenen Stand der Technik gilt dieses Gebiet der Riechstoffchemie als gut untersucht. Daher ist es besonders überraschend, dass auf dem Gebiet der Rosenriechstoffe nunmehr ein neuer, wertvoller Riechstoff gefunden werden konnte. Die Verbindung der Formel (I) verfügt über ganz eigenständige olfaktorische Eigenschaften, die sich deutlich von den bekannten Riechstoffen abheben und diese auch übertreffen. Die Eignung der erfindungsgemäß zu verwendenden Verbindung als Riech- oder Aromastoff für den Einsatz in der Riech- und Aromenindustrie war bislang nicht bekannt und ist überraschend.

Die Geruchsbeschreibungen aus eigenen Untersuchungen und bereits aus der Literatur (S. Arctander, Perfume and Flavor Materials, Vol. I und II, Montclair, N. J. 1969, Eigenverlag) bekannten strukturell verwandten Verbindungen lassen sich wie folgt zusammenfassen:
1. Cyclohexyl-Methanol: moderig, campherig, leicht minzig
2. 2,6,6-Trimethylcyclohex-2-enyl-Methanol: weich, blumig-süß, schwache Ausstrahlung
3. 2,6,6-Trimethylcyclohex-1-enyl-Methanol: minzig, frisch, leicht grün, eucalyptusartig
4. 2,2,6-Trimethylcyclohexyl-Methanol: moderig, coniferig, campherig

Die Geruchseigenschaften der erfindungsgemäß zu verwendenden Verbindung (5-Isopropyliden-2-Methyl-Cyclohexyl)-Methanol werden hingegen wie folgt beschrieben: sehr schöner strahlender Rosenriechstoff, interessante Duftnote in Richtung Pfingstrose, sehr natürlicher Geruchseindruck.

Die erfindungsgemäße Verbindung wird im Rahmen der erfindungsgemäßen Verwendung üblicherweise in sensorisch wirksamen Mengen eingesetzt. Häufig soll das erfindungsgemäß zu verwendende (5-Isopropyliden-2-MethylCyclohexyl)-Methanol mit anderen Riech- oder Aromastoffen gemischt werden.

Bei diesen weiteren Riech- oder Aromastoffen kann es sich um sonstige Riech- oder Aromastoffe handeln. Das Gewichtsverhältnis der Gesamtmenge an erfindungsgemäß zu verwendendem Riechstoff zu der Gesamtmenge an weiteren Riech- oder Aromastoffen liegt vorzugsweise im Bereich von 1: 1000 bis 1: 0,5.

Es ist besonders überraschend, dass die erfindungsgemäße Verbindung, insbesondere als Racemat, einen ausdrucksstarken Pfingstrosen-Geruch mit jeweils zusätzlichen, teilweise sehr komplexen und facettenre ichen Aspekten aufweist, denn aufgrund der Anwesenheit dieser weiteren zusätzlichen Aspekte unterscheidet sich die erfindungsgemäße Verbindung sehr deutlich von strukturell vergleichbaren und bekannten Substanzen (siehe oben), mit einer überraschenden Bandbreite an komplexen geruchlichen Schattierungen und vermittelt einen sehr komplexen und vielfältigen Geruchs- und Geschmackseindruck, der sonst nur durch komplexe Mischungen mehrerer Komponenten erreicht werden kann (wie beispielsweise durch ätherische Öle oder Kräuter- bzw. Gewürzmischungen).

In Mischungen mit anderen Riechstoffen vermag die erfindungsgemäße Verbindung der Formel (I) bereits in geringen Dosierungen die Intensität einer Riechstoffmischung zu verstärken und das Gesamtbild der Riechstoffmischung geruchlich abzurunden und zu harmonisieren sowie der Mischung mehr (Aus)Strahlung sowie Natürlichkeit zu verleihen.

Im Rahmen der erfindungsgemäßen Verwendung der oben genannten Verbindung der Formel (I) als Riech- oder Aromastoff (insbesondere mit blumigen, rosigen Noten) betrifft die vorliegende Erfindung auch die Verwendung zum Versehen von (a) Haaren, (b) Haut oder (c) textilen Fasern mit einem - insbesondere blumigen, rosigen Duft (hinsichtlich weiterer Geruchs- bzw. Geschmacksnoten s. oben). Die vorliegende Erfindung betrifft auch entsprechende Verfahren und (vorzugsweise tensidhaltige) Mischungen. Gemäß einem verwandten Aspekt betrifft die vorliegende Erfindung auch die Verwendung der oben genannten Verbindung der Formel (I) als Mittel zum Erhöhen des über einer tensidhaltigen wässrigen Lösung wahrgenommenen Geruches anderer Riechstoffe.

Im Zusammenhang mit der bevorzugten Verwendung zum Modifizieren und/oder Verstärken einer Geruchs- und/oder Geschmacksnote steht auch die Erkenntnis, dass die erfindungsgemäß zu verwendende Verbindung der Formel (I) hervorragend als sogenannte Booster (Verstärker; Enhancer) fungieren kann.

Die erfindungsgemäß zu verwendenden Verbindung der Formel (I) besitzt über ihre primären, geruchlichen Eigenschaften hinaus zusätzliche positive Sekundäreigenschaften, wie z. B. eine hohe Stabilität in sauren und alkalischen Medien, eine hohe Ausgiebigkeit, ein gutes Haftungsvermögen, eine hohe Substantivität und eine gute Booster-Wirkung.

Die Verbindung der Formel (I), als Racemat oder in Form eines oder mehrerer der Enantiomeren, kann insbesondere eingesetzt werden, um einer Riech- oder Aromastoffkomposition (Aus)Strahlung, Abrundung und/oder Harmonie zu verleihen und/oder vorhandene Geruchs- und/oder Geschmacksnoten zu verstärken.

Die vorliegende Erfindung betrifft auch Riechstoff- und Aromastoffmischungen sowie parfümierte oder aromatisierte Artikel umfassend
(a) (5-Isopropyliden-2-Methyl-Cyclohexyl)-Methanol (Formel I) 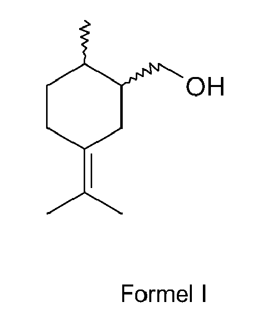 vorzugsweise in einer Menge, die ausreicht, eine Geruchs- oder Geschmacksnote des Typs strahlender Rosenriechstoff, interessante Duftnote in Richtung Pfingstrose, zu vermitteln, zu modifizieren und/oder zu verstärken
   sowie
(b) einen, zwei, drei oder mehr weitere Riech- oder Aromastoffe, bei denen es sich jeweils nicht um (5-Isopropyliden-2-Methyl-Cyclohexyl)-Methanol (Formel I) handelt, wobei der oder die weiteren Riech- oder Aromastoffe vorzugsweise einen blumigen, rosigen und/oder holzigen Geruch und/oder Geschmack vermitteln.

Die vorstehend mit Blick auf die erfindungsgemäßen Verwendungen beschriebenen bevorzugten Ausgestaltungen der Erfindung gelten entsprechend auch für erfindungsgemäße parfümierte und/oder aromatisierte Artikel, insbesondere die Angaben zu bevorzugten Gewichtsverhältnissen.

Vorzugsweise handelt es sich bei einem erfindungsgemäßen parfümierten oder aromatisierten Artikel um eine Riech- oder Aromastoffkomposition. Durch Kombination der Verbindung der Formel (I), mit einem, zwei, drei oder mehr weiteren Riech- oder Aromastoffen (mit vorzugsweise holzigem und/oder blumigem Geruch oder Geschmack) lassen sich neue Riech- oder Aromastoffkompositionen bilden. Auf diese Weise lassen sich besonders interessante und natürliche neue und originelle Duftnoten kreieren. Riechstoffe (als Komponente (b)), die zur Kombination vorteilhafterweise geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien genannt:
Extrakte aus natürlichen Rohstoffen wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-ÖI; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-ÖI; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E) - und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyl-dodecahydronaphtho[2,1-b]furan; 3a-Ethyl-6,6,9a-trimethyl-dodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Eine erfindungsgemäße Riech- oder Aromastoffkomposition lässt sich beispielsweise herstellen, indem eine Komponente (a), also (5-Isopropyliden-2-Methyl-Cyclohexyl)-Methanol als Racemat oder in Form eines oder mehrerer Enantiomeren, mit einer oder mehreren weiteren Riech- oder Aromastoffen (als Komponente (b)) vermischt wird. Die Komponente (a) wird dabei regelmäßig in einer Menge eingesetzt, die ausreicht, in der fertigen Komposition einen Geruchs- oder Geschmacksnote des Typs blumig, rosig zu vermitteln, zu modifizieren und/oder zu verstärken.

Eine erfindungsgemäße Riech- oder Aromastoffkompositionen umfasst vorzugsweise eine Gesamtmenge der Komponente (a) (also des Racemats bzw die Gesamtmenge aller Enantiomere der Formel (I)) im Bereich von 0,00001 bis 99,9 Gew.-%, vorzugsweise 0,001 bis 70 Gew.-% und besonders bevorzugt 0,01 bis 50 Gew.-%, bezogen auf die Gesamtmenge der Riech- oder Aromastoffkomposition.

Sofern die erfindungsgemäße Verbindung hauptsächlich eingesetzt wird, um einer Riech- oder Aromastoffkomposition mehr (Aus)Strahlung, Abrundung und/oder Harmonie zu verleihen und/oder bestimmte Noten zu verstärken, liegt die Gesamtmenge der Komponente (a) vorzugsweise vergleichsweise niedrig und besonders bevorzugt im Bereich von 0,01 bis 5 Gew.-%, bevorzugt im Bereich von 0,1 bis 2 Gew.-%, bezogen auf die Gesamtmenge der Riech- oder Aromastoffkomposition. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zu der Vermittlung der oben angegebenen Eigengeruchs- oder -geschmacksnoten.

Die Herstellung der erfindungsgemäßen Verbindung der Formel (I) kann durch entsprechende Anpassung an sich bekannter Reaktionen und Verfahren (Jerry March, Advanced Organic Chemistry: Reactions, Mechanisms and Structure, 4. Ed., John Wiley and Sons, 1992 und Richard C. Larock, Comprehensive Organic Transformations: A Guide to functional Group Preparations, VCH Verlag, 1989) erfolgen.

Zum Beispiel kann die erfindungsgemäßen Verbindung der Formel (I) über eine Prins-Reaktion aus Terpinolen (1) zu (4) und anschließender selektiver katalytischer Hydrierung hergestellt werden (siehe Formelschema).

Die selektive katalytische Hydrierung kann dabei unter dem Fachmann dem Prinzip nach bekannten Hydrierbedingungen (P.N. Rylander Catalytic Hydrogenation in Organic Synthesis, Academic Press, New York, 1979; Robert L. Augustine Heterogeneous Catalysis for the Synthetic Chemist, Marcel Dekker verlag, 1995) bei 20°-120°C und 10-50 bar, vorzugsweise zwischen 50-70°C und 20-30 bar Wasserstoff-Atmosphäre durchgeführt werden.

Alternativ kann die erfindungsgemäßen Verbindung der Formel (1) ausgehend von (5-Isopropyliden-2-Methyl-Cyclohexyl)-Carbaldehyd (2) (Herstellung durch Hydroformylierung gemäß DE 10205835 A1) via Reduktion, mit geeigneten Reduktionsmitteln wie z. B.: Natriumborhydrid, Lithiumaluminiumhydrid oder selektive katalytische Hydrierung unter, dem Fachmann dem Prinzip nach bekannten Hydrierbedingungen (P. N. Rylander und R. L. Augustine siehe oben), bei 50°-100°C und 20-50bar Wasserstoff-Atmosphäre hergestellt werden (siehe Formelschema). 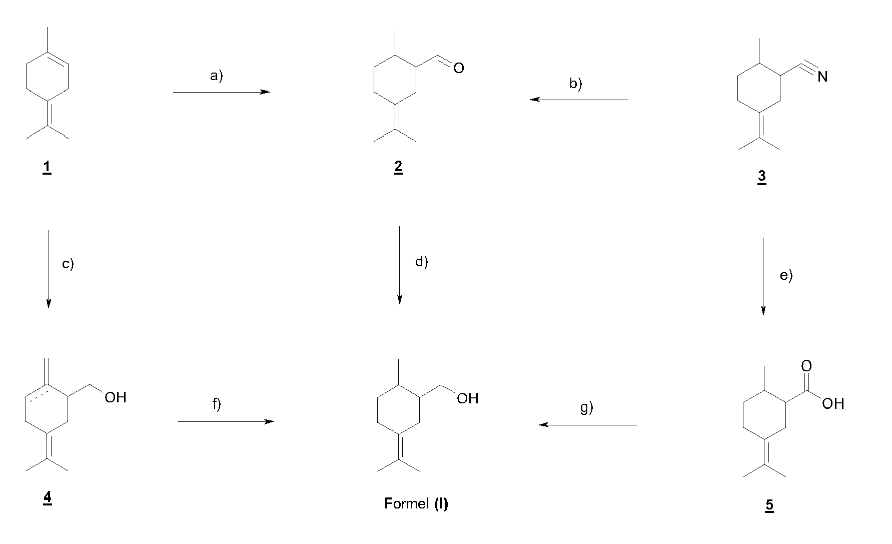 ***a)** Toluol, Triphenylphospin, Rhodium-2-Ethylhexanoat, CO*/*H2, 26MPa*/*130°C. **b)** DIBAL -H, THF. **c)** 1.Paraformaldehyd, Essigsäure*/*Acetanhydrid, 2. NaOH, MeOH, RF. **d)** Natriu m-borhydrid, Methanol. **e)** verd. Schwefelsäure, 130°C. **f)** Methanol, H2, Raney-Nickel, 50°C*/*20bar. **g)** Lithiumaluminiumhydrid, THF, RF.*

Eine weitere Möglichkeit zur Herstellung einer Verbindung der Formel (I) besteht in der Umwandlung der Nitrilfunktion von (5-Isopropyliden-2-Methyl-Cyclohexyl)-Carbonitril (3) mit verdünnter Schwefelsäure, konz. Salzsäure oder Natronlauge (Jerry March und Richard C. Larock, siehe oben), zu Carbonsäure (5) und anschließender Reduktion mit Komplexenhydriden wie z.B. Lithiumaluminiumhydrid (siehe Formelschema).

Ebenfalls kann die Verbindung der Formel (I) ausgehend von (3) durch Reduktion in Anlehnung an literaturbekannte Verfahren (Jerry March und Richard C. Larock, siehe oben) mit Diisopropylaluminiumhydrid zum (5-Isopropyliden-2-MethylCyclohexyl)-Carbaldehyd (2) und anschließender Reduktion, mit geeigneten Reduktionsmitteln hergestellt werden (siehe Formelschema).

Das bei den Reaktionen verwendete (5-Isopropyliden-2-Methyl-Cyclohexyl)-Carbonitril (3)

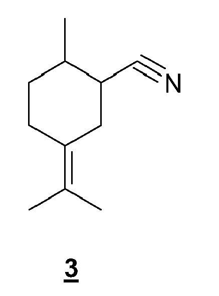

ist ebenfalls neu. Zur Herstellung der erfindungsgemäßen Verbindung der Formel (I) sind die Enantiomeren der Verbindung (3) und Mischungen zweier oder mehrerer der Enantiomeren der Verbindung (3) verwendbar. Die Verbindung der Formel (3) in Form ihres Enantiomerengemischs bzw. der Enantiomeren ist als Aroma- bzw. Riechstoff verwendbar, sie vermittelt bzw. vermitteln einen citrischen, nitrilischen Geruchseindruck.

Die Verbindung der Formel (3) als Enantiomerengemisch kann in Anlehung an literaturbekannte Verfahren (Jerry March Advanced Organic Chemistry: Reactions, Mechanisms and Structure, 4. Ed., John Wiley and Sons, 1992 und Richard C. Larock Comprehensive Organic Transformations: A Guide to functional Group Preparations, VCH Verlag, 1989) über folgendes Reaktionschema synthetisiert werden; einzelne Enantiomere können aus dem so erhaltenen Gemisch über chirale Trennmittel aufgereinigt werden: 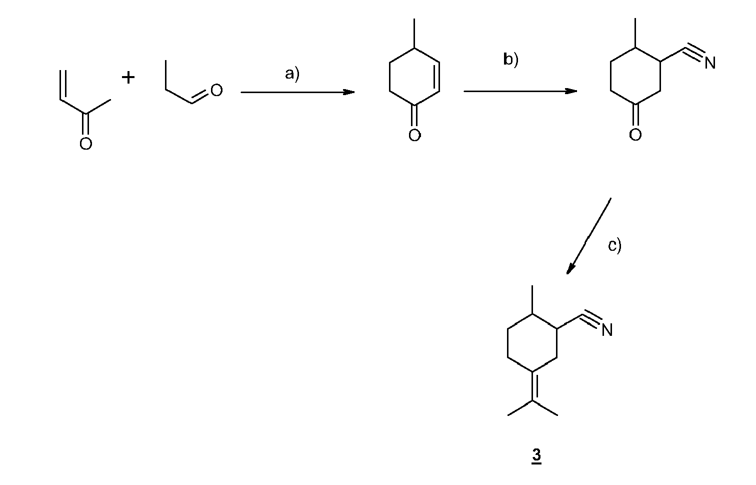 *a)1. Toluol, 0,2eq. Diethylamin, 50°C; 2. KOH 5%ig, Methanol, RF. b) NaCN, Ammoniumchlorid, DMF*/*Wasser, RF. c) NaH 60%ig, Isopropyltriphenyl-Phosphonium Jodid , THF, RF.*

Erfindungsgemäße Riech- oder Aromastoffkompositionen, welche eine (oder mehrere Enantiomere der) Verbindung der Formel (I) enthalten, können in flüssiger Form, unverdünnt oder mit einem Lösungsmittel verdünnt zur Parfümierung oder Aromatisierung eingesetzt werden. Geeignete Lösungsmittel hierfür sind z.B. Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin usw.

Des Weiteren können erfindungsgemäße Riech- oder Aromastoffkompositionen, welche die Verbindung der Formel (I) enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Erfindungsgemäße Riech- oder Aromastoffkompositionen, die die Verbindung der Formel (I) enthalten, können auch mikroverkapselt, sprühgetrocknet, als Einschluss-Komplexe oder als Extrusions-Produkte (d. h. erfindungsgemäße Artikel) vorliegen und in dieser Form z. B. einem zu parfümierenden oder aromatisierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- oder Aromastoffkompositionen zu erfindungsgemäßen Artikeln kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- oder Aromastoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Die Verbindung der Formel (I) und Riech- oder Aromastoffkompositionen, die die Verbindung der Formel (I) enthalten, können in konzentrierter Form, in Lösungen oder in oben beschriebener modifizierter Form verwendet werden für die Herstellung von erfindungsgemäßen parfümierten Artikeln wie z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und - lotionen, Gesichtscremes und -lotionen, Sonnenschutzcremes-und -lotionen, After-sun-cremes und -lotionen, Handcremes und -lotionen, Fußcremes und - lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die Verbindung der Formel (I) kann in aromatisierte oder zu aromatisierende Artikel eingearbeitet werden, insbesondere in der Ernährung, der Mundpflege oder dem Genuss dienende Zubereitungen.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßen Verbindung der Formel (I) sind diese Zubereitungen erfindungsgemäße Zubereitungen (als Beispiel erfindungsgemäßer Artikel).

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Erfindungsgemäße Zubereitungen (als Beispiele erfindungsgem äßer Artikel), enthaltend (5-Isopropyliden-2-Methyl-Cyclo-hexyl)-Methanol werden gemäß einer bevorzugten Ausgestaltung hergestellt, indem die Verbindung der Formel (I) als Substanz, als Lösung (z. B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen der Verbindung und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatineoder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung wird die Verbindung der Formel (I) zunächst mit einem oder mehreren Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass die Verbindung der Formel (I) verzögert von der Matrix freigegeben wird, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für die Mundpflege dienende Zubereitungen), die die Verbindung der Formel (I) enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für die Mundpflege dienende Zubereitungen), welche die Verbindung der Formel (I) enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugt können die erfindungsgemäßen Zubereitungen auch neben der erfindungsgemäßen Verbindung eine Aromakomposition enthalten, um den Geschmack und/oder Geruch der Zubereitung abzurunden und zu verfeinern. Ge- eignete (zusätzliche) Aromakompositionen enthalten z.B. synthetische, natürliche oder naturidentische Aroma-, Riech- und Geschmacksstoffe sowie geeignete Hilfs- und Trägerstoffe.

Nachfolgend wird die Erfindung anhand von Beispielen näher erläutert, ohne dass diese Beispiele den Schutzbereich der Ansprüche einschränken sollte. Sofern nicht anders angegeben, beziehen sich alle Angaben auf das Gewicht.

### Beispiel 1: Herstellung einer Verbindung der Formel (I) via Hydrierung

In einen 0,5 I Rührautoklav wurden 166 g (1 mol) (5-Isopropyliden-2-MethylCyclohexyl)-Carbaldehyd in 150 ml Ethanol mit 1,2 g Raney-Nickel vorgelegt. Anschließend wurde bei 60°C und einem Druck von 20 bar 8 Stunden unter Rühren bis zur Aufnahme von 1 Mol Wasserstoff hydriert. Danach wurde auf Raumtemperatur abgekühlt, die Reaktionslösung filtriert und eingeengt. Das Rohprodukt (165 g) wurde an einer 30 cm Vigreux-Kolonne im Vakuum fraktioniert.

Ausbeute:154,6 g (92% d. Th.) Sdp.: 110°-115°C/8 mbar

GC-Auswertung (20m ZB-WAX, Innendurchmesser 0,18µm / 60-9-220°C Kaltaufgabesystem)

### Beispiel 2: Herstellung einer Verbindung der Formel (I) via Reduktion

In einen 2 I Rührwerk wurden 166 g (1mol) (5-Isopropyliden-2-Methyl-Cyclohexyl)-Carbaldehyd in 600 ml Methanol unter Rühren und Kühlen bei 20°C mit einer Lösung aus 50 g Wasser, 200 mg NaOH und 12,7 g Natriumboranat versetzt. Danach wurde 4 h bei 20°C nachgerührt. Zur Aufarbeitung wurde das Methanol am Rotationsverdampfer entfernt, der Rückstand mit 100 g Toluol und 400 g Wasser versetzt. Nach Trennung der Phasen wurde die organische Phase einmal mit Wasser gewaschen und das Toluol entfernt. Rohausbeute: 170 g. Das Rohprodukt wurde an einer 30 cm Vigreux-Kolonne im Vakuum fraktioniert.

Ausbeute: 157,2 g (93,6% d. Th.) Sdp.: 110°-115°C/8 mbar

GC-Auswertung (20m ZB-WAX, Innendurchmesser 0,18µm / 60-9-220°C Kaltaufgabesystem)

Es wurden die spektroskopischen Daten von hergestellten E/Z-(5-Isopropyliden-2-Methyl-Cyclohexyl)-Methanol ermittelt. Die Daten sind nachfolgend angegeben.

### (5-Isopropyliden-2-Methyl-Cyclohexyl)-Methanol

¹H-NMR (CDCI₃, 400 MHZ, TMS= 0): 0,95(J= 6.5, 3H); 1,14(1H); 1,66(3H); 1,68 (3H); 2,59(1H); 2,71 (1H); 3,57(1H); 3,73(1H).

¹³C-NMR (CDCI₃, 100 MHz): 130,7(s), 121,0(s); 66,0(t), 47,3(d); 35,8(t), 33,3(d); 32,4(t), 29,5(t); 20,0(q), 19,7(qq).

MS: m/z (%) = 168 (67, M⁺), 137 (60), 135 (100), 107 (86), 95 (38), 93 (50), 81 (54), 79 (33).

MS: m/z (%) = 168 (66, M⁺), 137 (72), 135 (97), 107 (100), 95 (42), 93 (58), 81 (69), 79 (43).

### Beispiel 3: Parfümkomposition (Riechstoffkomposition)

| Bestandteil | Gew.-Teile |
|---|---|
| Agrumex LC / Cyclohexanol, 2-(1,1-dimethylethyl)-acetate | 10 |
| Amarocit® 10%ig in DPG / 6,6-Dimethoxy-2,5,5-trimethylhex-2-ene | 10 |
| Ambroxid krist. / Tetramethyldodecahydronaphthofuran | 10 |
| Basilikumöl | 10 |
| Calone 1951 10%ig in DPG / 7-methyl-benzodioxepin 3(4H)-one | 10 |
| Cedernholzöl | 10 |
| Cedrol krist. / | 50 |
| Citral 10%ig in DPG | 10 |
| Citonellol | 5 |
| Cumarin | 10 |
| Cyclogalbanat® 10%ig in DPG / Allyl (cyclohexyloxy) acetat | 15 |
| Dihydromyrcenol | 80 |
| Farenal® 10%ig in DPG / 2,6,10-Trimethylundec-9-enal | 5 |
| Galbex 10%ig in DPG / Undecatrien | 25 |
| Globalide® / Oxacyclohexadecen-2-one | 80 |
| Globanone® / Cyclohexadec-8-en-1-one | 40 |
| Hedion / 2-Pentyl-3-oxo-cyclopentaneacetic acidmethylester | 90 |
| Helional | 20 |
| Heliotropin | 5 |
| Hexenol cis-3 10%ig in DPG | 15 |
| Hexenylsalicylat cis-3 | 10 |
| Beta-Ionon | 5 |
| Iso E Super / Tetramethyl--octahydro-2-naphtalenylmethylketon | 180 |
| Isodamascon® 10%ig in DPG / Trimethylcyclohex-2-en-1-yl) but-2-en-1-one | 10 |
| Isomuscone (Cyclohexadecanon) | 20 |
| Isoraldein 70 / Methyljonon alpha | 20 |
| Ketamber 10%ig in TEC / 14,15-Bisnorlabdan, 8alpha,13;13,17-diepoxy- | 25 |
| Lavandinoel Grosso Nat. | 15 |
| Lilial / Isobutanal, 3-p-tert.-butylphenyl- | 20 |
| Linalool | 20 |
| Linalylacetat | 40 |
| Mandarinenoel brasil. grün | 50 |
| Timberol® / Cyclohexanepropanol, 2,2,6-trimethyl-alpha-propyl- | 40 |
| Vanillin | 5 |
| Veloutone 10%ig in DPG / Cyclopentanone, 2,2,5-trimethyl-5-pentyl- | 20 |
| Ysamber K® / Isolongifolanon-ethylenglycolketal | 10 |
| Gesamt | 1000 |

wobei der jeweilige INCI-Name bzw. die jeweilige chemische Bezeichnung dem üblichen Handelsnamen abgetrennt durch "/" nachgestellt ist.

### DPG: Dipropylenglycol, TEC = Triethylcitrat

Geruchsbeschreibung der Parfümkomposition ohne Zusatz von Verbindung der Formel I: holzig, blumig, herb

Nach Meinung der Parfümeure wird diese Parfümkomposition durch den Zusatz von 6 Gew.-% von Verbindung der Formel I aus Beispiel 2 frischer, strahlender, abgerundeter und harmonischer, wobei eine blumig-rosige Note hinzukommt und die blumigen Aspekte verstärkt werden. Die eingesetzte Verbindung der Formel I verleihen der Komposition durch ihren Eigengeruch sowie durch ihre modifizierende und verstärkende Wirkung (Booster-Wirkung) einen eigenen Charakter und verbinden die unterschiedlichen geruchlichen Elemente.

### Beispiel 4: Parfümkomposition (Riechstoffkomposition)

| | |
|---|---|
| Allylcyclohexylpropionat | 3.00 |
| Amylsalicylat | 2.00 |
| Benzylacetat | 64.00 |
| Citronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG / 1-Hydroxy-2-hydroxymethyl indan, methylene ether | 16.00 |
| Galaxolide 50%ig in DPG / Cyclopentan[g]-2-benzopyran, 1,3,4,6,7,8-hexahydro-4,6,6,7,8,8-hexamethyl- | 164.00 |
| Geraniol | 35.00 |
| Dihydromethyljasmonate / Cyclopentane acetic acid, 3-oxo-2-pentyl-, methyl ester | 6.00 |
| Heliotropin | 4.00 |
| Hexylzimtaldehyd | 121.00 |
| Vertocitral / 2,4-Dimethyltetrahydrobenzaldehyd | 4.00 |
| Hedion / Cyclopentane acetic acid, 3-oxo-2-pentyl-, methyl ester | 42.00 |
| Indol | 2.00 |
| Isobutylsalicylat | 6.00 |
| Lavandinoel Grosso Nat. | 6.00 |
| Acetylcedren / | 10.00 |
| Citronellylacetat | 190.00 |
| Linalool Synth. | 35.00 |
| Linalylacetat Synth. | 10.00 |
| Methylanthranilat 10%ig in DPG | 5.00 |
| Nerol | 10.00 |
| Orangenoel | 6.00 |
| Phantolide / Indan, 6-acetyl-1,1,2,3,3,5-hexamethyl- | 4.00 |
| Phenylacetaldehyddimethylacetal / 2-Phenylethan, 1,1-dimethoxy- | 6.00 |
| Phenylethylalkohol | 75.00 |
| Florol / Oxan, 4-hydroxy-2-isobutyl-4-methyl- | 6.00 |
| Sandelholzoel | 3.00 |
| Sandranol / 2-Buten-1-ol, 2-ethyl-4-(2,2,3-trimethyl-3-cyclopenten-1-yl)- | 16.00 |
| Trifernal / Butanal, 3-phenyl- | 2.00 |
| Tonalid / 7-Acetyl-1,1,3,4,4,6-hexamethyltetralin | 2.00 |
| | |
| Gesamt | 1000.00 |

### DPG: Dipropylenglycol

Geruchsbeschreibung der Parfümkomposition ohne Zusatz von Verbindung der Formel I: blumig, frisch.

Nach Meinung der Parfümeure erwacht diese Parfümkomposition durch den Zusatz von 10 Gew.-% von Verbindung der Formel I aus Beispiel 1 zu neuem Leben. Der Eindruck von Blumigkeit in Richtung Pfingstrose wird erheblich verstärkt. Die Komposition wirkt strahlender, abgerundeter und harmonischer, wobei eine natürliche Note hinzukommt. Die eingesetzte Verbindung der Formel I verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 5: Shampoo

Die Verbindung der Formel I aus Beispiel 1 wurde in einer Dosierung von 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| | |
| Cocamidopropylbetain (z.B. Dehyton K, Fa. Cognis Deutschland GmbH) | 2% |
| Natriumchlorid | 1,4% |
| Citronensäure | 1,3% |
| | |
| Phenoxyethanol, Methyl-, Ethyl-, Butyl-, | |
| und Propylparaben | 0,5% |
| Wasser | 82,8% |

Der pH-Wert der Shampoo-Grundmasse lag bei etwa 6. Hieraus wurden 100 ml einer 20 Gew.%-igen wässrigen Shampoo-Lösung hergestellt. In dieser Shampoo-Lösung wurden 2 Haarsträhnchen gemeinsam 2 Minuten lang gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

Geruchsbeschreibung jeweils: sehr schöner strahlender Rosengeruch, interessante Duftnote in Richtung Pfingstrose, sehr natürlicher Geruchseindruck.

### Beispiel 6: Weichspüler

Die Parfümkomposition aus Beispiel 3 (nach Zusatz von 3 Gew.-% von Verbindung Formel I aus Beispiel 2) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% (z.B. Rewoquat WE 18, Fa. Witco Surfactants GmbH) | 5,5% |
| Alkyldimethylbenzylammoniumchlorid, ca. 50% (z.B. Preventol R50, Fa. Bayer AG) | 0,2% |
| Farblösung, ca. 1%-ig | 0,3% |
| | |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% von Verbindung Formel I umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: blumig, rosig, interessante Duftnote, erinnert an Pfingstrose, sehr natürlicher Geruchseindruck Aspekte mit leichten süßen Untertönen, abgerundeter und harmonischer Geruchseindruck.

### Beispiel 7: Waschpulver

Die Parfümölkomposition aus Beispiel 4 (nach Zusatz von 6 Gew.-% von Verbindung Formel I aus Beispiel 1) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis | |
| (methylen)]]tetrakis-phosphonsäure, Natriumsalz) | |
| Optischer Aufheller | 0,2 % |
| Na-Sulfat | 6,5 % |
| Protease | 0,4 % |
| Natriumperborattetrahydrat | 22,0 % |
| TAED | 1,0% |

Zwei Stofflappen wurden mit 370 g einer 1%-igen wässrigen Waschpulverlauge auf Basis des 0,4 Gew.-% der Parfümölkomposition aus Beispiel 4 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: stark blumig, strahlend, und natürliche Note mit leichten süßen und holzigen Untertönen, abgerundeter und harmonischer Geruchseindruck.

## Patentansprüche

1. (5-Isopropyliden-2-Methyl-Cyclohexyl)-Methanol (Formel I) 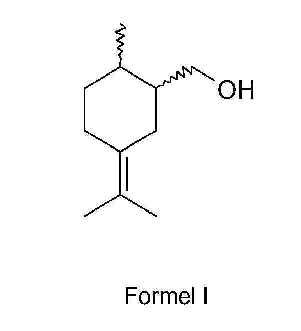 (1S, 2S)-(Isoproyliden-2-methyl-cyclohexyl)-1-methanol, (1S, 2R)-(Isoproyliden-2-methyl-cyclohexyl)-1-methanol, (1R, 2S)-(Isoproyliden-2-methyl-cyclohexyl)-1-methanol, (1R, 2R)-(Isoproyliden-2-methyl-cyclohexyl)-1-methanol, oder Mischung zweier oder mehrerer dieser Enantiomere.

2. Verwendung von (5-Isopropyliden-2-Methyl-Cyclohexyl)-Methanol (Formel I) 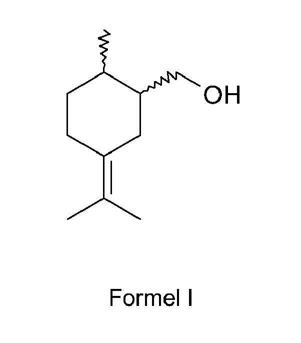 , von (1S, 2S)-(Isoproyliden-2-methyl-cyclohexyl)-1-methanol, (1S, 2R)-(Isoproyliden-2-methyl-cyclohexyl)-1-methanol, (1R, 2S)-(Isoproyliden-2-methylcyclohexyl)-1-methanol, (1R, 2R)-(Isoproyliden-2-methyl-cyclohexyl)-1-methanol, oder eines Gemisches zweier oder mehrerer der Entantiomeren der Formel (I) als Riech- oder Aromastoff.

3. Verwendung nach Anspruch 2 zum Vermitteln, Modifizieren und/oder Verstärken einer Geruchs- und/oder Geschmacksnote mit folgenden Aspekten: blumigen, rosig.

4. Riechstoff- oder Aromastoff-Mischung, umfassend
(a) 5-Isopropyliden-2-Methyl-Cyclohexyl)-Methanol (Formel I) 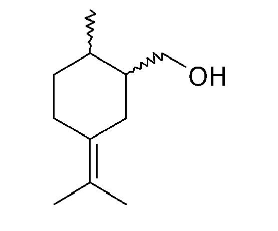 , (1S, 2S)-(Isoproyliden-2-methyl-cyclohexyl)-1-methanol, (1S, 2R)-(Isoproyliden-2-methyl-cyclohexyl)-1-methanol, (1R, 2S)-(Isoproyliden-2-methyl-cyclohexyl)-1-methanol, (1R, 2R)-(Isoproyliden-2-methylcyclohexyl)-1-methanol, oder eine Mischung zweier oder mehrerer dieser Enantiomeren, und
(b) einen, zwei, drei oder mehr weitere Riech- oder Aromastoffe, bei denen es sich jeweils nicht um die Verbindung der Formel (I) handelt, wobei der oder die weiteren Riech- oder Aromastoffe vorzugsweise einen holzigen und/oder blumigen Geruch und/oder Geschmack vermitteln.

5. Riechstoff- oder Aromastoffmischung nach Anspruch 4, **dadurch gekennzeichnet, dass** die Mischung eine Gesamtmenge an Verbindungen der Formel (I) von 0,00001 bis 99,9 Gew.-%, vorzugsweise 0,001 bis 70 Gew.-% und besonders bevorzugt 0,01 bis 50 Gew.-% umfasst, jeweils bezogen auf die Gesamtmenge an Riech- oder Aromastoffen.

6. Parfümierter und/oder aromatisierter Artikel, umfassend eine Riechstoff- oder Aromastoffmischung nach einem der Ansprüche 4 bis 5.

7. Artikel nach Anspruch 6, wobei der Artikel ausgewählt ist aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen, Kerzen, Lampenölen, Räucherstäbchen, Insektiziden, Repellentien und Treibstoffen.

8. Verfahren zum Vermitteln, Modifizieren und/oder Verstärken eines Geruchs oder Geschmacks eines Produktes mit einer oder sämtlichen der Noten blumig und rosig, umfassend das Zufügen einer sensorisch wirksamen Menge einer Verbindung der Formel (I):

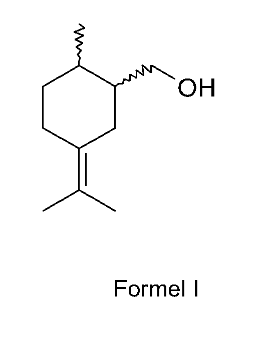

9. Verfahren zum Herstellen einer Verbindung der Formel (I) 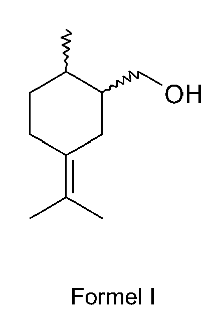 umfassend die Schritte:
(a) Durchführen einer Prins-Reaktion von Terpinolen (1) zu (4) und anschließender selektiver katalytischer Hydrierung: 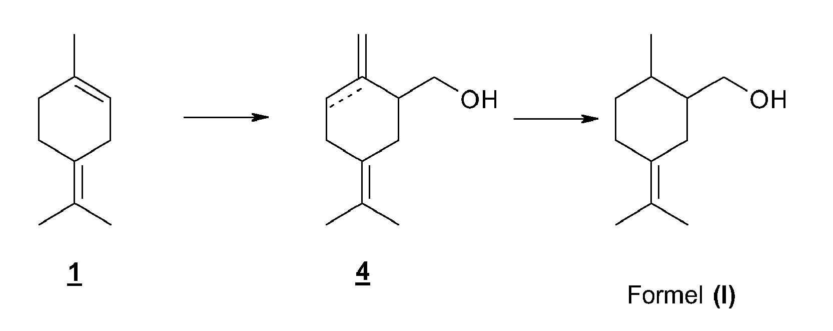
(b) Reduzieren von (5-Isopropyliden-2-Methyl-Cyclohexyl)-Carbaldehyd (2): 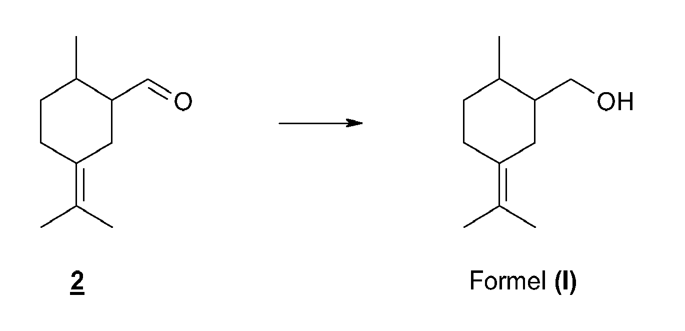
(c) Umwandeln von (1) zu (2) durch Hydroformylierung und anschließendes Durchführen von Schritt (b): 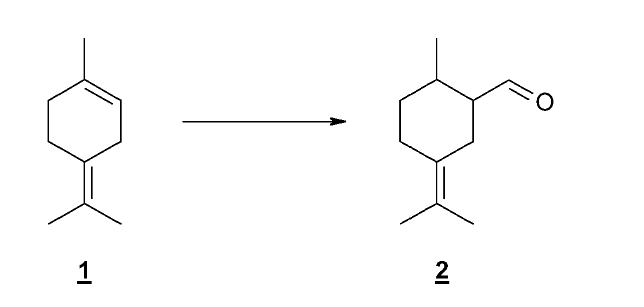
(d) Reduzieren von (5-Isopropyliden-2-Methyl-Cyclohexyl)-Carbonitril (3) zu (2) und anschließendes Durchführen von Schritt (b): 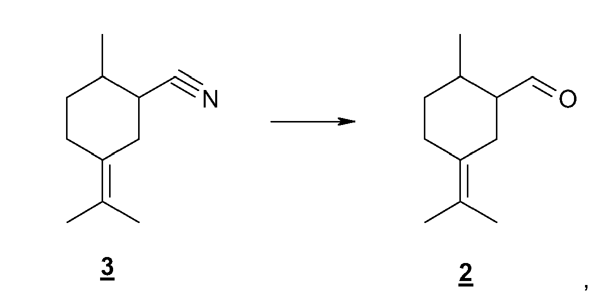 oder
(e) Umsetzen von (5-Isopropyliden-2-Methyl-Cyclohexyl)-Carbonitril (3) zu (5) und anschließende Reduktion von (5) zu (I): 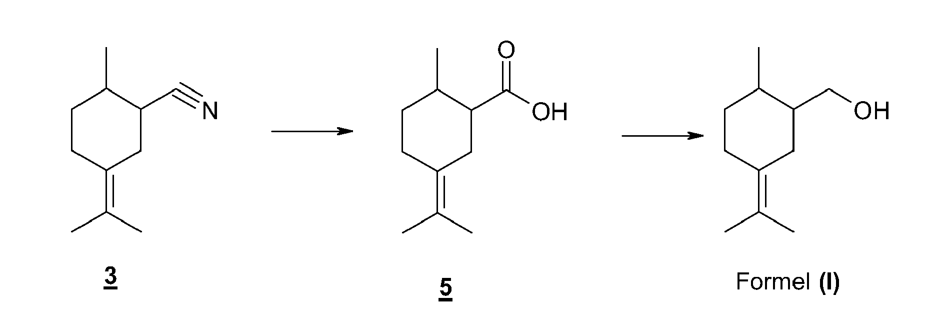 und optional nach Durchführen eines der genannte Schritte Aufreinigen eines oder mehrerer Enantiomeren der Formel (I).

10. (5-Isopropyliden-2-Methyl-Cyclohexyl)-Carbonitril der Formel (3): 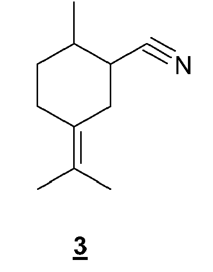 und dessen syn- und anti-Enantiomeren.

11. Verfahren zum Herstellen von (5-Isopropyliden-2-Methyl-Cyclohexyl)-Carbonitril der Formel (3), umfassend die Schritte:
a) Durchführen einer Michael-Addition mit anschließender Aldolkondensation,
b) Addition von HCN, und
c) Durchführen einer Wittig-Reaktion zum Erhalten von (5-Isopropyliden-2-Methyl-Cyclohexyl)-Carbonitril der Formel (3):
